# EUROPEAN PATENT SPECIFICATION

(11) **EP 2 716 277 B1**
(45) Date of publication and mention of the grant of the patent: **10.02.2016**
(21) Application number: 12792288.8
(22) Date of filing: 14.05.2012
(51) Int. Cl.: A61K 8/31, A61K 8/37, A61K 8/891, A61K 8/92, A61Q 1/04, A61Q 1/06

(54) **SOLID COSMETIC FOR LIPS**
FESTSTOFFKOSMETIKUM FÜR DIE LIPPEN
PRODUIT COSMÉTIQUE SOLIDE POUR LÈVRES

(30) Priority: 01.06.2011 JP 2011123757
(43) Date of publication of application: 09.04.2014
(73) Proprietor: Shiseido Company, Ltd., Tokyo 104-0061 (JP)
(72) Inventor: TOMITA, Noriko, Yokohama-shi Kanagawa 224-8558 (JP); SASADA, Kaori, Yokohama-shi Kanagawa 224-8558 (JP); IKEDA, Tomoko, Yokohama-shi Kanagawa 224-8558 (JP); KATAYAMA, Mika, Yokohama-shi Kanagawa 224-8558 (JP); WATANABE, Tomoko, Yokohama-shi Kanagawa 224-8558 (JP)
(74) Representative: Lippert, Stachow & Partner
(86) International application number: PCT/JP2012/062293
(87) International publication number: WO 2012/165130

(56) References cited:
- EP-A1- 2 298 273
- WO-A1-96/40044
- WO-A1-2005/097046
- WO-A1-2009/150852
- JP-A- 2005 255 626
- US-A1- 2005 226 832

## Description

### RELATED APPLICATIONS

This application claims the priority of Japanese Patent Application No. 2011-123757 filed on June 1, 2011.

### FIELD OF THE INVENTION

The present invention relates to a solid cosmetic for lips, and in particular, relates to a solid cosmetic for lips having excellent secondary adhesion resistance effect, excellent feeling in use, gloss durability, and excellent stability.

### BACKGROUND OF THE INVENTION

Conventional lip cosmetics have presented the problem of secondary adhesion, namely a lipstick is transferred onto a site contacted by a lip (for example, a cup) after the lipstick is applied to the lip. By contrast, lip cosmetics having so-called secondary adhesion resistance effect that causes little secondary adhesion have been developed.

For example, Patent Document 1 discloses a transfer-resistant cosmetic composition comprising: a volatile hydrocarbon solvent; a non-volatile silicone compound that can be dissolved or dispersed in the volatile hydrocarbon solvent; and non-volatile hydrocarbon oil that is dissolved in the volatile solvent and is incompatible with the non-volatile silicone compound, wherein the non-volatile hydrocarbon oil has a certain solubility parameter.

However, this transfer-resistant cosmetic composition has room for improvement in stability and gloss is insufficient. In addition, it takes some time until the onset of the secondary adhesion resistance effect after application. Since a volatile oil component is contained as an essential component, there is a problem in that some restrictions apply to the container.

Patent Document 2 discloses a lipstick composition having transfer resistance, comprising perfluoropolyether-type non-volatile oil and volatile oil, which are incompatible with each other. In this Patent Literature 2, oils are separated during application to a support to move onto a first composition.

However, the lipstick composition takes some time until the onset of the secondary adhesion resistance effect after application. Since a volatile oil component is contained as an essential component, there is a problem in that some restrictions apply to the container.

Patent Document 3 discloses a stick cosmetic having transfer resistance, comprising volatile oil and a silicone surfactant, wherein pigments are favorably dispersed.

However, this stick cosmetic has a large proportion of the volatile oil in the composition and thus has the disadvantage that its matte finish tends to provide a feeling of dryness on lips.

Patent Document 4 discloses a one-phase composition for lipsticks, comprising volatile oil and a silicone resin.

However, after evaporation of the volatile oil, this composition for lipsticks tends to cause a feeling of dryness over time, although it has improved transfer resistance. Moreover, a film of the resin remains on lips. The composition further has the following disadvantages that; it causes a filmy feeling and tightness, and the obtained adhesion is matte.

Patent Document 5 discloses an oil-in-oil emulsion composition comprising: continuous-phase oil comprising a silicone coating agent, volatile silicone oil, non-volatile silicone liquid oil, and an emulsifying agent; and dispersion-phase oil comprising ester oil and a coloring material, wherein the blending quantities of the continuous-phase oil and the dispersion-phase oil are at a dispersion-phase oil/(dispersion-phase oil and continuous-phase oil) ratio of 0.05 to 0.5.

However, it may be difficult to maintain for the oil-in-oil emulsion composition to maintain the temporal stability. In addition, it takes some time until the onset of the secondary adhesion resistance effect after application. Since a volatile oil component is contained as an essential component, there is a problem in that some restrictions apply to the container.
Patent literature 1: Japanese unexamined patent publication No. 2001-199846
Patent literature 2: International unexamined patent publication No. 96/40044
Patent literature 3: International unexamined patent publication No. 97/16157
Patent literature 4: Japanese unexamined patent publication No. H9-48709
Patent literature 5: Japanese unexamined patent publication No. 2000-53530

### DISCLOSURE OF THE INVENTION

### PROBLEM TO BE SOLVED BY THE INVENTION

The present invention was made in view of the above-described conventional art. An object of the invention is to provide a solid cosmetic for lips that has excellent secondary adhesion resistance effect, gloss durability after application, and excellent stability.

### MEANS TO SOLVE THE PROBLEM

The present inventors have diligently studied; as a result, the present inventors have found that a solid cosmetic for lips having an excellent secondary adhesion resistance effect, without losing a gloss, from immediately after application can be obtained by using a combination of specific oils component and by blending wax.

That is, the solid cosmetic for lips of the present invention is characterized by comprising the following (a) to (d):
(a) 5 to 30 mass % of hydrogenated polyisobutene,
(b) 30 to 70 mass % of one or more kinds of methyl phenyl silicones that separate from (a) when mixed therewith at 25 °C,
(c) 0.5 to 15 mass % of oil that separates from both (a) and (b) when mixed therewith at 25 °C, and
(d) 4 to 12 mass % of wax.

In the cosmetic, it is preferable to comprise (e) a coloring material.

In the cosmetic, it is preferable that component (e) is virtually dispersed in component (c).

In the cosmetic, it is preferable that component (b) comprises one or more kinds of methyl phenyl silicones selected from the group consisting of trimethyl pentaphenyl trisiloxane, diphenyl dimethicone, diphenylsiloxyphenyl trimethicone, and phenyl trimethicone.

In the cosmetic, it is preferable that component (c) comprises one or more kinds of oils selected from the group consisting of dipentaerythrityl hexahydroxystearate, castor oil, glyceryl monoisostearate, polyglyceryl isostearate wherein the addition mole number of glycerin is 4 to 10 and the number of isostearic acid residues is 1 to 4, PEG/PPG-36/41 dimethyl ether, PEG/polybutylene glycol-52/32 dimethyl ether, pentaerythrityl tetra(ethylhexanoate/benzoate), and pentaerythrityl tetra(behenate/benzoate/ethylhexanoate).

In the cosmetic, it is preferable that component (a) further comprises a dimer dilinoleic acid ester.

In the cosmetic, it is preferable to comprise one or more kinds of component (f) selected from the group consisting of olefin oligomers, neopentyl glycol dicaprate, glyceryl tri-2-ethylhexanoate, sorbitan sesquiisostearate, propylene glycol monostearate, cetyl PEG/PPG-10/1 dimethicone, diglyceryl diisostearate, glyceryl diisostearate, pentaerythrityl tetraethylhexanoate, squalane, liquid paraffin, trimethylolpropane triisostearate, trimethylolpropane triethylhexanoate, diisostearyl malate, and cetyl ethylhexanoate.

The solid cosmetic for lips of the present invention preferably comprises the following (a) to (d):
(a) 5 to 30 mass % of hydrogenated polyisobutene,
(b) 30 to 70 mass % of trimethyl pentaphenyl trisiloxane.
(c) 0.5 to 15 mass % of one or more kinds of oils selected from the group consisting of dipentaerythrityl hexahydroxystearate, castor oil, and polyglyceryl isostearate wherein the addition mole number of glycerin is 4 to 10 and the number of isostearic acid residues is 1 to 4, and
(d) 4 to 12 mass % of wax.

The solid cosmetic for lips of the present invention preferably comprises the following (a) to (d):
(a) 5 to 30 mass % of hydrogenated polyisobutene,
(b) 30 to 70 mass % of diphenyl dimethicone,
(c) 0.5 to 15 mass % of one or more kinds of oils selected from the group consisting of dipentaerythrityl hexahydroxystearate, castor oil, polyglyceryl isostearate wherein the addition mole number of glycerin is 4 to 10 and the number of isostearic acid residues is 1 to 4, PEG/PPG-36/41 dimethyl ether, and pentaerythrityl tetra(behenate/benzoate/ethylhexanoate), and
(d) 4 to 12 mass % of wax.

### EFFECT OF THE INVENTION

A solid cosmetic for lips having excellent secondary adhesion resistance effect from immediately after the application, gloss durability, and good stability, can be obtained by blending the specific amounts of (a) hydrogenated polyisobutene, (b) one or more kinds of methyl phenyl silicones that separate from (a) when mixed therewith at 25 °C, (c) oil that separates from both (a) and (b) when mixed therewith at 25 °C, and (d) wax.

### BRIEF DESCRIPTION OF THE DRAWINGS

Fig. 1 is the illustration of the mechanism for the solid cosmetic for lips of the present invention (A) during production, (B) at the time of product formation (solidification), and (C) at the time of application on the lip.
Fig. 2 is a photograph of the sample, of the present invention, obtained by mixing components (a) to (c) and a coloring material (Test Example 1-1) after dissolving them with heating, centrifuging, and allowing to stand still.

### BEST MODE FOR CARRYING OUT THE INVENTION

The solid cosmetic for lips of the present invention comprises (a) hydrogenated polyisobutene, (b) one or more kinds of methyl phenyl silicones that separate from (a) when mixed therewith at 25 °C, (c) oil that separates from both (a) and (b) when mixed therewith at 25 °C, and (d) wax.

In the following, each component is described in detail.

### ((a) Hydrogenated polyisobutene)

Component (a) is the oil component which is insoluble in component (b) which is continuous phase oil component. Component (a) has a higher affinity to the lip than component (b) and easily adheres to the lip.

The average molecular weight of the hydrogenated polyisobutene is preferably 1000 to 2650.

As component (a), in addition to hydrogenated polyisobutene, a dimer dilinoleic acid ester can be blended.

As dimer dilinoleic acid ester, phytosteryl/behenyl dimer dilinoleate is preferable.

It is necessary that the blending quantity of component (a) is 5 to 30 mass % of the total amount of the cosmetic. The blending quantity of component (a) is preferably 10 mass % or higher and more preferably 12 mass % or higher. If it is less than 5 mass %, the secondary adhesion resistance effect isn't be attained because the cosmetic isn't separated upon application. The blending quantity of component (a) is preferably 25 mass % or lower and more preferably 20 mass % or lower. Also, if it exceeds 30 mass %, the secondary adhesion resistance effect isn't attained because it is difficult for the cosmetic to separate upon application.

### ((b) Methyl phenyl silicone)

In the present invention, (b) methyl phenyl silicone separates when mixed with (a) hydrogenated polyisobutene at 25 °C.

The (b) methyl phenyl silicone blended in the solid cosmetic for lips of the present invention can be one kind or a mixture of two or more kinds.

Here, the presence or absence of "separation" was measured under the following conditions.

### (Measurement condition)

(a) and (b) were used in the ratio ((a):(b) = 1:1 (mass ratio)) and heated to 90 °C. After the mixture was mixed with stirring, it was allowed to stand at 25 °C. When the boundary was uniformly separated into two layers, it was denoted "separated". When it was a translucent state or a transparently miscible state without a boundary, it was denoted "not separated".

When two kinds or more of methyl phenyl silicones are used as the component (b), the presence or absence of separation depends upon their blending ratio. Therefore, it is necessary to check the presence or absence of separation in light of the blending ratio of the component (b).

Examples of methyl phenyl silicones include trimethyl pentaphenyl trisiloxane, diphenyl dimethicone, diphenylsiloxyphenyl trimethicone, and phenyl trimethicone.

As methyl phenyl silicone blended in the solid cosmetic for lips, trimethyl pentaphenyl trisiloxane is preferable.

As a commercial trimethyl pentaphenyl trisiloxane, methyl phenyl silicone PH-1555 (180 mm²/s (25 °C), manufactured by Dow Corning Toray Co., Ltd.) and methyl phenyl silicone FZ3156 (165 mm²/s (25 °C), manufactured by Dow Corning Toray Co., Ltd.) can be listed.

As a commercial diphenyl dimethicone, silicone KF54 (400 mm²/s (25 °C), manufactured by Shin-Etsu Chemical Co., Ltd.), silicone KF50-300CS (270 to 330 mm²/s (25 °C), manufactured by Shin-Etsu Chemical Co., Ltd.), and silicone KF-54HV (5000 mm²/s (25 °C), manufactured by Shin-Etsu Chemical Co., Ltd.) can be listed.

As a commercial diphenylsiloxyphenyl trimethicone, silicone KF56 (14 mm²/s (25 °C), manufactured by Shin-Etsu Chemical Co., Ltd.) can be listed.

As a commercial phenyl trimethicone, silicone SH556 (22 mm²/s (25 °C), manufactured by Dow Corning Toray Co., Ltd.) can be listed.

It is necessary that the blending quantity of component (b) is 30 to 70 mass % of the total amount of the cosmetic. The blending quantity of component (b) is preferably 40 mass % or higher. If it is less than 30 mass %, the secondary adhesion resistance effect isn't attained because it is difficult for the cosmetic to separate upon application. The blending quantity of component (b) is preferably 60 mass % or lower. If it exceeds 70 mass %, the secondary adhesion resistance effect isn't attained because the blending quantity of other components is decreased.

### ((c) Oil)

Component (c) is oil that separates from both (a) and (b) when mixed therewith at 25 °C. Component (c) has a higher affinity to the lip than component (b) and easily adheres to the lip.

Component (c) blended in the solid cosmetic for lips of the present invention can be one kind or a mixture of two or more kinds.

Here, the presence or absence of "separation" was measured under the following conditions.

### (Measurement condition)

(a) and (c) were used in the ratio ((a):(c) = 1:1 (mass ratio)) and heated to 90 °C. After the mixture was mixed with stirring, it was allowed to stand at 25 °C. When the boundary was uniformly separated into two layers, it was denoted "separated". When it was a translucent state or a transparently miscible state without a boundary, it was denoted "not separated".

Examples of (c) oils include dipentaerythrityl hexahydroxystearate, castor oil, glyceryl monoisostearate, polyglyceryl isostearate wherein the addition mole number of glycerin is 4 to 10 and the number of isostearic acid residues is 1 to 4, PEG/PPG-36/41 dimethyl ether, PEG/polybutylene glycol-52/32 dimethyl ether, pentaerythrityl tetra(ethylhexanoate/benzoate), and pentaerythrityl tetra(behenate/benzoate/ethylhexanoate).

Among them, it is preferable to comprise dipentaerythrityl hexahydroxystearate.

It is necessary that the blending quantity of component (c) is 0.5 to 15 mass % of the total amount of the cosmetic. The blending quantity of component (c) is preferably 1 mass % or higher. If it is less than 0.5 mass %, the secondary adhesion resistance effect and gloss feeling with time are poor. The blending quantity of component (c) is preferably 10 mass % or lower. If it exceeds 15 mass %, the color unevenness takes place.

In the solid cosmetic for lips of the present invention, when only hydrogenated polyisobutene as component (a) and only trimethyl pentaphenyl trisiloxane as component (b) are used, one or more kinds of oils selected from the group consisting of dipentaerythrityl hexahydroxystearate, castor oil, and polyglyceryl isostearate, wherein the addition mole number of glycerin is 4 to 10 and the number of isostearic acid residues is 1 to 4, can be used as component (c).

Such component (c) is oil that separates from both (a) hydrogenated polyisobutene and (b) trimethyl pentaphenyl trisiloxane when mixed therewith at 25 °C.

In the solid cosmetic for lips of the present invention, when only hydrogenated polyisobutene as component (a) and only diphenyl dimethicone as component (b) are used, one or more kinds of oils selected from the group consisting of dipentaerythrityl hexahydroxystearate, castor oil, polyglyceryl isostearate wherein the addition mole number of glycerin is 4 to 10 and the number of isostearic acid residues is 1 to 4, PEG/PPG-36/41 dimethyl ether, and pentaerythrityl tetra(behenate/benzoate/ethylhexanoate), can be used as component (c).

Such component (c) is oil that separates from both (a) hydrogenated polyisobutene and (b) diphenyl dimethicone when mixed therewith at 25 °C.

By using the above-described components (a) to (c), when the solid cosmetic for lips of the present invention is applied on the lip, component (a), component (b), and component (c) immediately separate by the contact of the cosmetic and the lip, component (a) and component (c) adhere on the lip, and low-viscosity component (b) separates into the surface layer; thus the secondary adhesion resistance effect is realized. When such a cosmetic contacts a material, only transparent component (b) adheres. In addition, because a large amount of component (b) is present, component (b) again separates into the surface layer after the contact of the material and the lip. Accordingly, the solid cosmetic for lips of the present invention can realize the secondary adhesion resistance effect over a long time.

### ((d) Wax)

(d) Wax blended in the solid cosmetic for lips of the present invention is not limited in particular as long as it can be normally blended for cosmetics.

In the present invention, a solid cosmetic for lips, wherein (b) methyl phenyl silicone that is a liquid oil component and normally has lower viscosity than components (a) and (c) is the external phase, can be obtained by blending (d) wax. That is, it is preferable that the wax used in the present invention is compatible with the methyl phenyl silicone.

Examples of (d) waxes include carnauba wax, candelilla wax, polyethylene wax, beeswax, ceresin, microcrystalline wax, solid paraffin, and Japan wax.

It is necessary that the blending quantity of component (d) is 4 to 12 mass % of the total amount of the cosmetic. The blending quantity of component (d) is preferably 6 mass % or higher. If it is less than 4 mass %, the solidification is difficult. The blending quantity of component (d) is preferably 11 mass % or lower. If it exceeds 12 mass %, the spreadability becomes heavy and the gloss is lost.

In the solid cosmetic for lips of the present invention, in addition to the above-described essential components (a) to (d), the components normally used in lip cosmetics can be blended as optional component.

In the solid cosmetic for lips of the present invention, it is preferable to blend (e) a coloring material.

Such coloring materials can be powdery or lake-like (oil-containing state) so far as they are coloring materials normally used in lip cosmetics. They can be inorganic pigments, organic pigments, or pearlescent agents. Inorganic pigments, organic pigments, and pearlescent agents are all more wettable to the dispersed-phase oil component (in particular, component (c)) than to the continuous phase oil component (component (b)). Accordingly, the coloring material spontaneously moves into the dispersed-phase oil component. Therefore, the coloring material is held in dispersed-phase oil component when the cosmetic is applied and it is present in the inner side of component (b) of surface layer; thus the secondary adhesion is difficult to take place.

Thus, it is preferable in the solid cosmetic for lips of the present invention that component (e) is virtually dispersed in component (c). It is more preferable that 80 mass % or higher of component (e) of the total amount of the coloring material is dispersed in component (c).

The blending quantity of the coloring material is preferably 1 mass % or higher and more preferably 5 mass % or higher of the total amount of the cosmetic. If it is too small, it may be difficult to feel the secondary adhesion resistance effect. The blending quantity of the coloring material is preferably 15 mass % or lower and more preferably 10 mass % or lower.

It is preferable in the solid cosmetic for lips of the present invention to blend one or more kinds of component (f) selected from the group consisting of olefin oligomers, neopentyl glycol dicaprate, glyceryl tri-2-ethylhexanoate, sorbitan sesquiisostearate, propylene glycol monostearate, cetyl PEG/PPG-10/1 dimethicone, diglyceryl diisostearate, glyceryl diisostearate, pentaerythrityl tetraethylhexanoate, squalane, liquid paraffin, trimethylolpropane triisostearate, trimethylolpropane triethylhexanoate, diisostearyl malate, and cetyl ethylhexanoate.

Component (f) is highly compatible with components (a) to (c) at a high temperature (90 °C). By blending such an oil, a stable solid cosmetic for lips wherein non-compatible components (a), (b), and (c) are blended can be easily produced.

When component (f) is blended, the blending quantity is preferably 1 mass % or higher and more preferably 3 mass % or higher of the total amount of the cosmetic. If it is less than 1 mass %, the cosmetic may be separated because the compatibility of bulk may be poor. The blending quantity of component (d) is preferably 20 mass % or lower and more preferably 15 mass % or lower. If it exceeds 20 mass %, the secondary adhesion resistance effect may not be attained because the component (b) may not separate upon application to the lips.

In the solid cosmetic for lips of the present invention, in addition to the above-described components, the components normally used in lip cosmetics (for example, oil other than the above-described oils, powder, polymer compound, moisturizer, perfume, antioxidant agent, preservative, beauty component, and the like) can be blended so far as the effect of the present invention is not undermined.

It is preferable that the solid cosmetic for lips of the present invention is constituted so that the separation does not take place throughout the entire production process and the state of one homogeneous phase is maintained.

The solid cosmetic for lips of the present invention can be applied to lipsticks, lip glosses, lip bases, overcoats for lipsticks, lip creams, and the like. In particular, a solid lipstick is preferable.

### EXAMPLES

The present invention will be further described in the following examples. However, the invention is not limited by these examples. Unless otherwise specified, the blending quantity of each component will be expressed in mass %.

The present inventors have found in the past that a solid cosmetic for lips, wherein (a) hydrogenated polyisobutene and a large amount of (b) one or more kinds of methyl phenyl silicones that separate from (a) when mixed therewith at 25 °C are solidified by wax, is excellent in the secondary adhesion resistance effect.

That is, when applied on the lip, component (b) with low viscosity and low affinity with the lip separates into the surface layer, and component (a) stays in the inner layer (lip surface). Because the coloring material has high affinity with component (a), the coloring material is enclosed in the inner layer. Thus, when the cosmetic for lips attaches to a cup, only component (b) in which no coloring material is blended adheres to the cup. Because component (b) is present in a large amount, component (b) seeps out, by the contact of a cup and the lip, from the cosmetic for lips. Therefore, in the above-described system, the excellent secondary adhesion resistance effect can be achieved for a long time.

Based on this knowledge, the conditions necessary to further improve the secondary adhesion resistance effect were investigated.

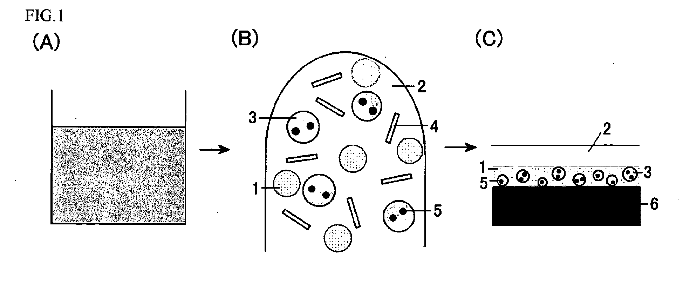

The present inventors contemplated, in anticipating the excellent secondary adhesion resistance effect, a solid cosmetic for lips that shows a behavior shown in Fig. 1(C) on the lip. According to Fig. 1(C), in the further inner layer than component (a), which is in the inner layer on the lip, a component which encloses the coloring material is present.

In order to produce such a solid cosmetic for lips, it is necessary that all the components do not separate and they are in a mixed (or fine dispersion) state (Fig. 1(A)) in the high-temperature production process, and it is considered that the state shown in Fig. 1(B) is taken when solidification takes place by cooling.

That is, a component having a higher affinity with the coloring material is necessary in addition to the above-described system, and an oil that separates from both component (a) and component (b) and has very low compatibility with component (b) is considered necessary.

In order to find such an oil based on the above consideration, the present inventors mixed oil components in the blending compositions, listed in the below Table 1, wherein component (a), component (b), a coloring material, and various additional oils were blended, and evaluated each sample based on the below-described evaluation criteria (1). The results are shown in Table 1.

The blending quantity in Table 1 is expressed in parts by mass. In the blending compositions in Table 1, the mixed component (b) solution separated from (a) when mixed therewith at 25 °C.

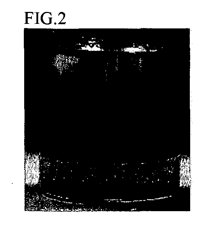

A photograph of the sample of Test Example 1-1 evaluated by evaluation (1) is shown in Fig. 2. This photograph was taken by placing the sample on a white desk.

### Evaluation (1): Evaluation test of the affinity to the coloring material

The mixed oil component was centrifuged and allowed to stand, and it was evaluated by the below-described evaluation criteria. When the oil component was semisolid oil, the mixed oil component was centrifuged after dissolution by heating and evaluated by a similar method.

### (Evaluation criteria)

A*: Almost all coloring material is dispersed in the additional oil.
A: Although the coloring material is dispersed in the (a) hydrogenated polyisobutene, most coloring material is dispersed in the additional oil.
B*: A similar amount of coloring material is dispersed in the (a) hydrogenated polyisobutene and in the additional oil.
B: Although the coloring material is dispersed in the additional oil, most coloring material is dispersed in the (a) hydrogenated polyisobutene.
C: Almost all coloring material is dispersed in the (a) hydrogenated polyisobutene.

**[Table 1]**

| Test Example | | 1-1 | 1-2 | 1-3 | 1-4 | 1-5 | 1-6 | 1-7 | 1-8 |
|---|---|---|---|---|---|---|---|---|---|
| (a) | Hydrogenated polyisobutene ( 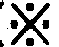 1) | 1 | 1 | 1 | 1 | 1 | 1 | 1 | 1 |
| (b) | Trimethyl pentaphenyl trisiloxane ( 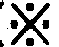 2) | 0.5 | 0.5 | 0.5 | 0.5 | 0.5 | 0.5 | 0.5 | 0.5 |
| | Diphenyl dimethicone ( 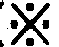 3) | 0.5 | 0.5 | 0.5 | 0.5 | 0.5 | 0.5 | 0.5 | 0.5 |
| | Dipentaerythrityl hexahydroxystearate ( 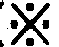 4) | 1 | - | - | - | - | - | - | - |
| | Polyglyceryl-5 triisostearate | - | 1 | - | - | - | - | - | - |
| | Glyceryl monoisostearate | - | - | 1 | - | - | - | - | - |
| | Castor oil | - | - | - | 1 | - | - | - | - |
| | PEG/Polybutylene glycol-52/32 dimethyl ether | - | - | - | - | 1 | - | - | - |
| | Macadamia nut oil polyglyceryl-6 esters behenate ( 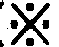 5) | - | - | - | - | - | 1 | - | - |
| | Phytosteryl macadamia nut fatty acid ( 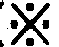 6) | - | - | - | - | - | - | 1 | - |
| | Vaseline | - | - | - | - | - | - | - | 1 |
| | Coloring material | 0.1 | 0.1 | 0.1 | 0.1 | 0.1 | 0.1 | 0.1 | 0.1 |
| Evaluation (1): Affinity to the coloring material | | A* | A* | A* | A* | A* | B* | B* | B* |

| | | | | | | | | | |
|---|---|---|---|---|---|---|---|---|---|
| 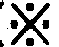 1: Deodorizing polybutene P (manufactured by NIKKO RICA CORPORATION) 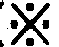 2: Methyl phenyl silicone PH-1555 (manufactured by Dow Corning Toray Co., Ltd.) 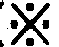 3: Silicone KF54 (manufactured by Shin-Etsu Chemical Co., Ltd.) 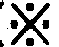 4: COSMOL 168M (manufactured by Nisshin OilliO Group, Ltd.) 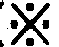 5: S-Face VL-211 (manufactured by Sakamoto Yakuhin Kogyo Co., Ltd.) 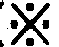 6: YOFCO-MAS (manufactured by Nippon Fine Chemical) | | | | | | | | | |

From Table 1, it is found that the layer in which the coloring material is dispersed varies depending upon the kind of blended additional oil.

According to Test Examples 1-1 to 1-5, especially when dipentaerythrityl hexahydroxystearate, polyglyceryl-5 isostearate, glyceryl monoisostearate, castor oil, or PEG/polybutylene glycol-52/32 dimethyl ether was blended as the additional oil, the coloring material was dispersed in the additional oil.

Next, samples (solid lipsticks) of the blending compositions, listed in Table 2, in which an additional oil listed in Table 1 was actually blended were produced by the conventional method. Each sample was evaluated based on the below evaluation criterion (2). The results are shown in Table 2.

In the blending compositions in Table 2, the mixed component (b) solution separated from component (a) when mixed therewith at 25 °C.

### Evaluation (2): Evaluation test of the secondary adhesion resistance effect

The actual usability test by 10 professional panelists was carried out. The five-level sensory evaluation (scoring) of the secondary adhesion resistance effect upon application to the lip was based on the below-described scoring criteria. The determination was by the score average value based on the below-described evaluation criteria.

### (Score)

5 points: very excellent
4 points: excellent
3 points: ordinary
2 points: poor
1 point: very poor

### (Evaluation criteria)

S: The score average value is 4.5 points or higher and less than 5 points.
A*: The score average value is 4 points or higher and less than 4.5 points.
A: The score average value is 3.5 points or higher and less than 4 points.
B: The score average value is 2.5 points or higher and less than 3.5 points.
C: The score average value is 1 point or higher and less than 2.5 points.

The examples listed with "-" in the table had poor stability, and the secondary adhesion resistance effect could not be measured.

**[Table 2]**

| Test Example | | 2-1 | 2-2 | 2-3 | 2-4 | 2-5 | 2-6 | 2-7 | 2-8 | 2-9 |
|---|---|---|---|---|---|---|---|---|---|---|
| (a) | Hydrogenated polyisobutene ( 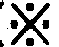 1) | 23.5 | 17.5 | 17.5 | 17.5 | 17.5 | 17.5 | 17.5 | 17.5 | 17.5 |
| (b) | Trimethyl pentaphenyl trisiloxane ( 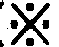 2) | 45 | 45 | 45 | 45 | 45 | 45 | 45 | 45 | 45 |
| | Diphenyl dimethicone ( 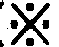 3) | 12.5 | 12.5 | 12.5 | 12.5 | 12.5 | 12.5 | 12.5 | 12.5 | 12.5 |
| | Dipentaerythrityl hexahydroxystearate ( 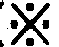 4) | - | 6 | - | - | - | - | - | - | - |
| | Polyglyceryl-5 triisostearate | - | - | 6 | - | - | - | - | - | - |
| | Glyceryl monoisostearate | - | - | - | 6 | - | - | - | - | - |
| | Castor oil | - | - | - | - | 6 | - | - | - | - |
| | PEG/Polybutylene glycol-52/32 dimethyl ether | - | - | - | - | - | 6 | - | - | - |
| | Macadamia nut oil polyglyceryl-6 esters behenate ( 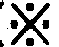 5) | - | - | - | - | - | - | 6 | - | - |
| | Phytosteryl macadamia nut fatty acid ( 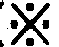 6) | - | - | - | - | - | - | - | 6 | - |
| | Vaseline | - | - | - | - | - | - | - | - | 6 |
| | Wax | 9 | 9 | 9 | 9 | 9 | 9 | 9 | 9 | 9 |
| | Coloring material | 10 | 10 | 10 | 10 | 10 | 10 | 10 | 10 | 10 |
| Evaluation (2): Secondary adhesion resistance effect | | A* | S | S | S | S | S | B | B | A |

From Table 2, it was clarified that the samples of Test Examples 2-2 to 2-6, wherein an additional oil having high affinity to the coloring material, shown in Table 1, was blended, were excellent in the secondary adhesion resistance effect.

Next, the properties of the oil that has a high affinity to the coloring material, represented by dipentaerythrityl hexahydroxystearate, polyglyceryl-5 isostearate, glyceryl monoisostearate, castor oil, or PEG/polybutylene glycol-52/32 dimethyl ether, were investigated.

The compatibility of the additional oil, used in Table 1, with component (a) or component (b) was evaluated by the below-described evaluation method (evaluation (3)). The results are shown in Table 3.

### Evaluation (3): Evaluation test of the separation state

Component (a) or (b) and additional oil were used in the ratio (component (a) or (b): additional oil = 1:1 (mass ratio)) and heated to 90 °C. After the mixture was mixed with stirring, it was allowed to stand at 25 °C. When the boundary was uniformly separated into two layers, it was denoted "separation". When it was a translucent state or a transparently miscible state without a boundary, it was denoted "uniform.".

**[Table 3]**

| | Evaluation (3): Separation state (a) | Evaluation (3): Separation state (b) |
|---|---|---|
| Dipentaerythrityl hexahydroxystearate ( 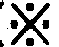 4) | Separation | Separation |
| Polyglyceryl-5 triisostearate | Separation | Separation |
| Glyceryl monoisostearate | Separation | Separation |
| Castor oil | Separation | Separation |
| PEG/Polybutylene glycol-52/32 dimethyl ether | Separation | Separation |
| Macadamia nut oil polyglyceryl-6 esters behenate ( 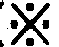 5) | Uniform | Uniform |
| Phytosteryl macadamia nut fatty acid ( 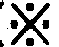 6) | Uniform | Uniform |
| Vaseline | Uniform | Separation |

From Table 3, it was clarified that dipentaerythrityl hexahydroxystearate, polyglyceryl-5 isostearate, glyceryl monoisostearate, castor oil, and PEG/polybutylene glycol-52/32 dimethyl ether are oils that separate from component (a) and component (b).

According to Test Examples 2-7 to 2-9, when macadamia nut oil polyglyceryl-6 esters behenate, phytosteryl macadamia nut fatty acid, or petrolatum was blended, a prominent secondary adhesion resistance effect could not be realized. This can be considered because when these oils are mixed with component (a) and/or component (b), the oil in which the coloring material is dispersed does not separate and dissolves in layer (a) and/or layer (b) without showing the behavior shown in Fig. 1(C).

Accordingly, in the solid cosmetic for lips of the present invention, it is necessary to comprise (a) hydrogenated polyisobutene, (b) one or more kinds of methyl phenyl silicones that separate from (a) when mixed therewith at 25 °C, (c) oil that separates from both (a) and (b) when mixed therewith at 25 °C, and (d) wax.

Next, component (c) was further investigated. The present inventors evaluated the compatibility of PEG/PPG-36/41 dimethyl ether and pentaerythrityl tetra(behenate/benzoate/ethylhexanoate) with component (a) or component (b) by the above-described evaluation method (evaluation (3)). Then, samples (solid lipsticks) blended each of these oils with the blending compositions shown in Table 4 were produced by the ordinary method. Each sample was evaluated based on the above evaluation criterion (2). The results are shown in Table 4.

In the blending compositions in Table 4, the mixed component (b) solution separated from component (a) when mixed therewith at 25 °C.

**[Table 4]**

| Test Example | | 4-1 | 4-2 | 4-3 |
|---|---|---|---|---|
| (a) | Hydrogenated polyisobutene ( 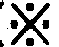 1) | 15.8 | 15.8 | 15.8 |
| (b) | Diphenyl dimethicone ( 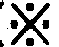 3) | 57.4 | 57.4 | 57.4 |
| (c) | Dipentaerythrityl hexahydroxystearate ( 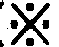 4) | 9 | - | - |
| | PEG/PPG-36/41 dimethyl ether | - | 9 | |
| | Pentaerythrityl tetra (behenate/benzoate/ethylhexanoate) ( 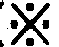 7) | - | - | 9 |
| (d) | Wax | 7 | 7 | 7 |
| | Coloring material | 10.8 | 10.8 | 10.8 |
| Evaluation (3): Separation state (a) | | Separation | Separation | Separation |
| Evaluation (3): Separation state (b) | | Separation | Separation | Separation |
| Evaluation (2): Secondary adhesion resistance effect | | S | S | S |

| | | | | |
|---|---|---|---|---|
| 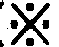 7: SALACOS P-B822 (manufactured by Nisshin OilliO Group, Ltd.) | | | | |

According to Table 4, PEG/PPG-36/41 dimethyl ether and pentaerythrityl tetra(behenate/benzoate/ethylhexanoate) are oils that separate from both component (a) and component (b).

A solid cosmetic for lips in which such an oil was blended was excellent in the secondary adhesion resistance effect.

Accordingly, as component (c) blended in the solid cosmetic for lips, PEG/PPG-36/41 dimethyl ether and pentaerythrityl tetra(behenate/benzoate/ethylhexanoate) are preferably used other than dipentaerythrityl hexahydroxystearate, polyglyceryl-5 triisostearate, glyceryl monoisostearate, castor oil, and PEG/polybutylene glycol-52/32 dimethyl ether.

Moreover, as a result of the investigation by the present inventors, it is clarified that polyglyceryl isostearate wherein the addition mole number of glycerin is 4 to 10 and the number of isostearic acid residues is 1 to 4 and pentaerythrityl tetra(ethylhexanoate/benzoate) can be also used as component (c).

Next, component (a) was investigated. The present inventors produced samples (solid lipsticks) with the blending compositions shown in Table 5 by the ordinary method. Each sample was evaluated based on the above evaluation criterion (2). The results are shown in Table 5.

In the blending compositions in Table 5, the mixed component (b) solution separated from component (a) when mixed therewith at 25 °C.

**[Table 5]**

| Test Example | | 5-1 | 5-2 | 5-3 |
|---|---|---|---|---|
| (a) | Hydrogenated polyisobutene ( 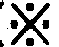 1) | 24.8 | 15.8 | 5 |
| | Phytosteryl/behenyl dimer dilinoleate ( 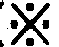 8) | - | - | 10.8 |
| (b) | Trimethyl pentaphenyl trisiloxane ( 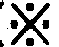 2) | 28.7 | 28.7 | 28.7 |
| | Diphenyl dimethicone ( 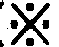 3) | 28.7 | 28.7 | 28.7 |
| (c) | Dipentaerythrityl hexahydroxystearate ( 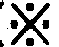 4) | - | 9 | 9 |
| (d) | Wax | 7 | 7 | 7 |
| | Coloring material | 10.8 | 10.8 | 10.8 |
| Evaluation (2): Secondary adhesion resistance effect | | A* | S | S |

| | | | | |
|---|---|---|---|---|
| 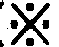 8: Plandool-PB (manufactured by Nippon Fine Chemical) | | | | |

From Test Example 5-3, it is seen that when a part of hydrogenated polyisobutene, which is component (a) of the present invention, is substituted by phytosteryl/behenyl dimer dilinoleate, which is a dimer dilinoleic acid ester, a solid cosmetic for lips excellent in the secondary adhesion resistance effect can be obtained.

Accordingly, in the solid cosmetic for lips of the present invention, a dimer dilinoleic acid ester can be used as component (a) in addition to hydrogenated polyisobutene.

Next, the other components were investigated.

The present inventors produced samples (solid lipsticks) with the blending compositions shown in Table 6 by the ordinary method. Each sample was evaluated based on the above evaluation criterion (2) and below evaluation criteria (4) to (8). The results are shown in Table 6.

In the blending compositions in Table 6, the mixed component (b) solution separated from component (a) when mixed therewith at 25 °C.

### Evaluation (4): Evaluation test of the sample (bulk) state

A bulk sample was vertically cut, and the surface state was determined by the below-described evaluation criteria.

### (Evaluation criteria)

A*: There is no aggregation of the coloring material, and the cut surface is smooth and uniform.
A: There is no aggregation of the coloring material, and the cut surface is almost uniform.
B: There is aggregation of the coloring material, and the cut surface is not smooth.
C: There is aggregation of the coloring material, and the cut surface is phase-separated.

### Evaluation (5) to (8): Evaluation tests for feeling in use (light spreadability/feeling of fitting/feeling of gloss/color appearance)

The actual usability test by 10 professional panelists was carried out. The five-level sensory evaluation (scoring) of the feeling in use (light spreadability/feeling of fitting/feeling of gloss/color appearance) upon application to the lip was based on the below-described scoring criteria. The determination was by the score average value based on the below-described evaluation criteria.

### (Score)

5 points: very excellent
4 points: excellent
3 points: ordinary
2 points: poor
1 point: very poor

### (Evaluation criteria)

S: The score average value is 4.5 points or higher and less than 5 points.
A*: The score average value is 4 points or higher and less than 4.5 points.
A: The score average value is 3.5 points or higher and less than 4 points.
B: The score average value is 2.5 points or higher and less than 3.5 points.
C: The score average value is 1 point or higher and less than 2.5 points.

**[Table 6]**

| Test Example | | 6-1 | 6-2 | 6-3 | 6-4 | 6-5 |
|---|---|---|---|---|---|---|
| (a) | Hydrogenated polyisobutene ( 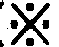 1) | 10.6 | 15.6 | 15.6 | 15.1 | 20.1 |
| (b) | Trimethyl pentaphenyl trisiloxane ( 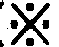 2) | 36.4 | 36.4 | 36.4 | 36.4 | 36.4 |
| | Diphenyl dimethicone ( 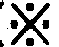 3) | 10 | 10 | 10 | 10 | 10 |
| | Diphenylsiloxyphenyl trimethicone ( 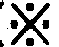 9) | 15 | 10 | 5 | - | - |
| (c) | Dipentaerythrityl hexahydroxystearate ( 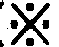 4) | - | 5 | 5 | 5 | 5 |
| (d) | Wax | 7 | 7 | 7 | 7.5 | 7.5 |
| | Coloring material | 13.5 | 13.5 | 13.5 | 13.5 | 13.5 |
| | Liquid paraffin | 5 | - | - | - | - |
| | Olefin oligomers | - | - | 5 | 10 | - |
| | Neopentyl glycol dicaprate | - | - | - | - | 5 |
| | Sorbitan sesquiisostearate | 2.5 | 2.5 | 2.5 | 2.5 | 2.5 |
| Evaluation (2): Secondary adhesion resistance effect | | A* | S | S | S | S |
| Evaluation (4): Bulk state | | A* | A | A* | A* | A |
| Evaluation (5): Light spreadability | | B | B | A* | S | A |
| Evaluation (6): Feeling of fitting | | A | A | A* | A* | A |
| Evaluation (7): Feeling of gloss | | B | A* | A* | A* | B |
| Evaluation (8): Color appearance | | B | B | A* | A* | B |

| | | | | | | |
|---|---|---|---|---|---|---|
| 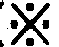 9: Silicone KF56 (manufactured by Shin-Etsu Chemical Co., Ltd.) | | | | | | |

The secondary adhesion resistance effect was improved by blending component (c). Test Examples 6-3 to 6-5, wherein an olefin oligomer or neopentyl glycol dicaprate was also blended, were excellent in the feeling in use.

In particular, Test Examples 6-3 and 6-4, wherein an olefin oligomer was blended, were also excellent in the bulk state.

As a result of the investigation by the present inventors, it has become clear that the compounds represented by olefin oligomers and neopentyl glycol dicaprate are highly compatible components with components (a) to (c) at a high temperature (90°C) during production.

Besides olefin oligomers and neopentyl glycol dicaprate, the examples of such components include, glyceryl tri-2-ethylhexanoate, sorbitan sesquiisostearate, propylene glycol monostearate, cetyl PEG/PPG-10/1 dimethicone, diglyceryl diisostearate, glyceryl diisostearate, pentaerythrityl tetraethylhexanoate, squalane, liquid paraffin, trimethylolpropane triisostearate, trimethylolpropane triethylhexanoate, diisostearyl malate, and cetyl ethylhexanoate. It is preferable to blend the above components as component (f) of the solid cosmetic for lips of the present invention.

Next, the blending quantity of component (c) was investigated. The present inventors produced samples (solid lipsticks) with the blending compositions shown in Table 7 by the ordinary method. Each sample was evaluated by the above-described evaluation criteria (2) and the below-described evaluation criteria (9). For the evaluation criteria (10) (gloss feeling with time), the evaluation was conducted according to the evaluation tests, for the feeling in use, of the above-described evaluation criteria (5) to (8). The results are shown in Table 7.

In the blending compositions in Table 7, the mixed component (b) solution separated from component (a) when mixed therewith at 25 °C.

### Evaluation (9): Evaluation test of the color unevenness

The color unevenness when a sample was applied on the lip was evaluated based on the below-described evaluation criteria.

### (Evaluation criteria)

A*: A color unevenness is not observed.
A: A slight color unevenness is observed.
B: A color unevenness is observed.
C: Much color unevenness is observed.

**[Table 7]**

| Test Example | | 7-1 | 7-2 | 7-3 | 7-4 | 7-5 |
|---|---|---|---|---|---|---|
| (a) | Hydrogenated polyisobutene ( 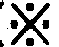 1) | 10 | 10 | 10 | 10 | 10 |
| | Phytosteryl/behenyl dimer dilinoleate ( 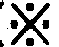 8) | 12.5 | 12.5 | 12.5 | 10 | 10 |
| (b) | Trimethyl pentaphenyl trisiloxane ( 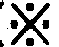 2) | 48.29 | 46.83 | 36.8 | 18.8 | - |
| | Diphenyl dimethicone ( 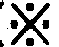 3) | 1.3 | 2.45 | 7 | 15 | 20 |
| | Diphenylsiloxyphenyl trimethicone ( 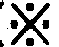 9) | 10 | 10 | 10 | 10 | 17.6 |
| (c) | Polyglyceryl-5 triisostearate | 0.25 | 0.5 | 4.9 | 15 | 20 |
| (d) | Lip wax | 4.4 | 4.4 | 4.4 | 4.4 | 4.4 |
| | Candelilla wax | 1.2 | 1.2 | 1.2 | 1.2 | 1.2 |
| | Coloring material | 9 | 9 | 9 | 9 | 9 |
| | Pearlescent agent | 1 | 1 | 1 | 1 | 1 |
| | Decamethylcyclopentasiloxane | 2 | 2 | 2 | 2 | 2 |
| | Dynamite glycerin | 0.06 | 0.12 | 1.2 | 3.6 | 4.8 |
| Evaluation (2): Secondary adhesion resistance effect | | A* | A* | S | S | S |
| Evaluation (9): colour unevenness | | A* | A* | A* | A* | B |
| Evaluation (10): Gloss feeling with time | | B | A* | A* | A* | A* |

It is seen from Table 7 that the gloss feeling with time was poor in Test Example 7-1, wherein the blending quantity of component (c) was small.

It is also seen that color unevenness was observed in Test Example 7-5 wherein the blending quantity of component (c) was large.

Accordingly, it is necessary that the blending quantity of component (c) blended in the solid cosmetic for lips of the present invention is 0.5 to 15 mass %.

Hereinafter, formulation examples of the solid cosmetic for lips of the present invention will be illustrated. It is to be understood that the present invention is not limited by these formulation examples. Each of the solid cosmetic for lips of Formulation Example had excellent secondary adhesion resistance effect, gloss durability, excellent feeling in use, and excellent stability.

### Formulation Example 1: Lipstick

| | |
|---|---|
| Hydrogenated polyisobutene ( 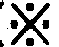 1) | 14.1 mass % |
| Trimethyl pentaphenyl trisiloxane ( 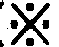 2) | 36.4 |
| Diphenyl dimethicone ( 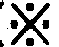 3) | 10 |
| Dipentaerythrityl hexahydroxystearate ( 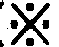 4) | 5 |
| Wax | 7.5 |
| Coloring material | 9.1 |
| Pearlescent agent | 5.4 |
| Olefin oligomers | 10 |
| Sorbitan sesquiisostearate | 2.5 |

### Formulation Example 2: Lipstick

| | |
|---|---|
| Hydrogenated polyisobutene ( 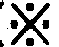 1) | 10.9 mass % |
| Phytosteryl/behenyl dimer dilinoleate ( 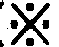 8) | 12.5 |
| Trimethyl pentaphenyl trisiloxane ( 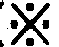 2) | 36.46 |
| Diphenyl dimethicone ( 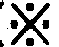 3) | 5 |
| Diphenylsiloxyphenyl trimethicone ( 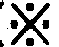 9) | 9.18 |
| Polyglyceryl-5 triisostearate | 4.9 |
| Lip wax | 4.15 |
| Candelilla wax | 1.1 |
| Coloring material | 7.1 |
| Pearlescent agent | 5.41 |
| Decamethylcyclopentasiloxane | 2 |
| Dynamite glycerin | 1.2 |
| Isostearic acid | 0.1 |

### Formulation Example 3: Lipstick

| | |
|---|---|
| Hydrogenated polyisobutene ( 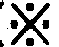 1) | 5 mass % |
| Phytosteryl/behenyl dimer dilinoleate ( 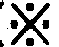 8) | 10.55 |
| Trimethyl pentaphenyl trisiloxane ( 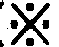 2) | 25.58 |
| Diphenyl dimethicone ( 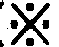 3) | 25 |
| Dipentaerythrityl hexahydroxystearate ( 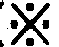 4) | 9 |
| Wax | 5 |
| Candelilla wax | 0.5 |
| Coloring material | 7.65 |
| Pearlescent agent | 3.22 |
| Olefin oligomers | 5 |
| Glyceryl tri-2-ethylhexanoate | 3.5 |

### DESCRIPTION OF THE NUMERALS

1: (a) Hydrogenated polyisobutene
2: (b) Methyl phenyl silicone
3: An additional oil (component (c))
4: (d) Wax
5: (e) A coloring material
6: A lip

## Claims

1. A solid cosmetic for lips comprising the following (a) to (d):
(a) 5 to 30 mass % of hydrogenated polyisobutene,
(b) 30 to 70 mass % of one or more kinds of methyl phenyl silicones that separate from (a) when mixed therewith at 25 °C,
(c) 0.5 to 15 mass % of oil that separates from both (a) and (b) when mixed therewith at 25 °C, and
(d) 4 to 12 mass % of wax.

2. The solid cosmetic for lips according to claim 1, further comprising (e) a coloring material in the cosmetic.

3. The solid cosmetic for lips according to claim 2, wherein component (e) is virtually dispersed in component (c).

4. The solid cosmetic for lips according to any one of claims 1 to 3, wherein component (b) comprises one or more kinds of methyl phenyl silicones selected from the group consisting of trimethyl pentaphenyl trisiloxane, diphenyl dimethicone, diphenylsiloxyphenyl trimethicone, and phenyl trimethicone.

5. The solid cosmetic for lips according to any one of claims 1 to 4, wherein component (c) comprises one or more kinds of oils selected from the group consisting of dipentaerythrityl hexahydroxystearate, castor oil, glyceryl monoisostearate, polyglyceryl isostearate wherein the addition mole number of glycerin is 4 to 10 and the number of isostearic acid residues is 1 to 4, PEG/PPG-36/41 dimethyl ether, PEG/polybutylene glycol-52/32 dimethyl ether, pentaerythrityl tetra(ethylhexanoate/benzoate), and pentaerythrityl tetra(behenate/benzoate/ethylhexanoate).

6. The solid cosmetic for lips according to any one of claims 1 to 5, wherein component (a) further comprises a dimer dilinoleic acid ester.

7. The solid cosmetic for lips according to any one of claims 1 to 6, further comprising one or more kinds of component (f) selected from the group consisting of olefin oligomers, neopentyl glycol dicaprate, glyceryl tri-2-ethylhexanoate, sorbitan sesquiisostearate, propylene glycol monostearate, cetyl PEG/PPG-10/1 dimethicone, diglyceryl diisostearate, glyceryl diisostearate, pentaerythrityl tetraethylhexanoate, squalane, liquid paraffin, trimethylolpropane triisostearate, trimethylolpropane triethylhexanoate, diisostearyl malate, and cetyl ethylhexanoate.

8. A solid cosmetic for lips comprising the following (a) to (d):
(a) 5 to 30 mass % of hydrogenated polyisobutene,
(b) 30 to 70 mass % of trimethyl pentaphenyl trisiloxane.
(c) 0.5 to 15 mass % of one or more kinds of oils selected from the group consisting of dipentaerythrityl hexahydroxystearate, castor oil, and polyglyceryl isostearate wherein the addition mole number of glycerin is 4 to 10 and the number of isostearic acid residues is 1 to 4, and
(d) 4 to 12 mass % of wax.

9. A solid cosmetic for lips comprising the following (a) to (d):
(a) 5 to 30 mass % of hydrogenated polyisobutene,
(b) 30 to 70 mass % of diphenyl dimethicone,
(c) 0.5 to 15 mass % of one or more kinds of oils selected from the group consisting of dipentaerythrityl hexahydroxystearate, castor oil, polyglyceryl isostearate wherein the addition mole number of glycerin is 4 to 10 and the number of isostearic acid residues is 1 to 4, PEG/PPG-36/41 dimethyl ether, and pentaerythrityl tetra(behenate/benzoate/ethylhexanoate), and
(d) 4 to 12 mass % of wax.

## Patentansprüche

1. Feststoffkosmetikum für Lippen, umfassend (a) bis (d) wie folgt:
(a) 5 bis 30 Gew.-% hydrogenisiertes Polyisobuten,
(b) 30 bis 70 Gew.-% einer oder mehrerer Arten von Methylphenylsilikonen, die sich von (a) abspalten, wenn sie bei 25°C mit diesem gemischt werden,
(c) 0,5 bis 15 Gew.-% Öl, das sich sowohl von (a) als auch (b) abspaltet, wenn es bei 25°C mit diesen gemischt wird, und
(d) 4 bis 12 Gew.-% Wachs.

2. Feststoffkosmetikum für Lippen gemäß Anspruch 1, ferner umfassend (e) einen Farbstoff in dem Kosmetikum.

3. Feststoffkosmetikum für Lippen gemäß Anspruch 2, wobei Komponente (e) in Komponente (c) praktisch dispergiert ist.

4. Feststoffkosmetikum für Lippen gemäß einem der Ansprüche 1 bis 3, wobei Komponente (b) eine oder mehrere Arten von Methylphenylsilikonen enthält, ausgewählt aus der Gruppe bestehend aus Trimethylpentaphenyltrisiloxan, Diphenyldimethicon, Diphenylsiloxyphenyltrimethicon und Phenyltrimethicon.

5. Feststoffkosmetikum für Lippen gemäß einem der Ansprüche 1 bis 4, wobei Komponente (c) eine oder mehrere Arten von Ölen enthält, ausgewählt aus der Gruppe bestehend aus Dipentaerythrityl Hexahydroxystearat, Rizinusöl, Glycerylmonoisostearat, Polyglycerylisostearat, wobei die Molzahl der Addition von Glycerin 4 bis 10 beträgt und die Anzahl von isostearischen Säureresten 1 bis 4 beträgt, PEG/PPG-36/41 Dimethylether, PEG/Polybutylenglykol-52/53 Dimethylether, Pentaerythrityltetra(ethylhexanoat/benzoat) und Pentaerythrityltetra(behenat/benzoat/ethylhexanoat).

6. Feststoffkosmetikum für Lippen gemäß einem der Ansprüche 1 bis 5, wobei Komponente (a) ferner einen dimeren Dilinolsäureester enthält.

7. Feststoffkosmetikum für Lippen gemäß einem der Ansrprüche 1 bis 6, ferner umfassend eine oder mehrere Arten von Komponente (f), ausgewählt aus der Gruppe bestehend aus Olefinoligomeren, Neopentylglykoldicaprat, Glyceryl-tri-2-ethylhexanoat, Sorbitansesquiisostearat, Polypropylenglykolmonostearat, Cetyl PEG/PPG-10/1 Dimethicon, Diglyceryldiisostearat, Glyceryldiisostearat, Pentaerythrityltetraethylhexanoat, Squalan, flüssiges Paraffin, Trimethylolpropantriisostearat, Trimethylolpropantriethylhexanoat, Diisostearylmalat und Cetylethylhexanoat.

8. Feststoffkosmetikum für Lippen, umfassend (a) bis (d) wie folgt:
(a) 5 bis 30 Gew.-% hydrogenisiertes Polyisobuten,
(b) 30 bis 70 Gew.-% Trimethylpentaphenyltrisiloxan,
(c) 0,5 bis 15 Gew.-% einer oder mehrere Arten von Ölen, ausgewählt aus der Gruppe bestehend aus Dipentaerythrityl Hexahydroxystearat, Rizinusöl und Polyglycerylisostearat, wobei die Molzahl der Addition von Glycerin 4 bis 10 beträgt und die Anzahl von isostearischen Säureresten 1 bis 4 beträgt, und
(d) 4 bis 12 Gew.-% Wachs.

9. Feststoffkosmetikum für Lippen, umfassend (a) bis (d) wie folgt:
(a) 5 bis 30 Gew.-% hydrogenisiertes Polyisobuten,
(b) 30 bis 70 Gew.-% Diphenyldimethicon,
(c) 0,5 bis 15 Gew.-% einer oder mehrere Arten von Ölen, ausgewählt aus der Gruppe bestehend aus Dipentaerythrityl Hexahydroxystearat, Rizinusöl, Polyglycerylisostearat, wobei die Molzahl der Addition von Glycerin 4 bis 10 beträgt und die Anzahl von isostearischen Säureresten 1 bis 4 beträgt, PEG/PPG-36/41 Dimethylether und Pentaerythrityl(tetra)benehat/benzoat/ethylhexanoat) und
(d) 4 bis 12 Gew.-% Wachs.

## Revendications

1. Produit cosmétique solide pour lèvres, comprenant (a) à (d) suivants:
a) 5 à 30 % en poids de polyisobutène hydrogéné,
b) 30 à 70% d'un ou plusieurs types de silicones de méthyle phényle qui se détachent du (a) lorsqu'ils sont mélangées avec celui-ci à 25°,
c) 0,5 à 15% en poids d'huile qui se détache de deux (a) et (b) lorsqu'elle est mélangée avec ceux-ci à 25°, et
d) 4 à 12% en masse de cire.

2. Produit cosmétique solide pour lèvres selon la revendication 1, en outre comprenant (e) un colorant dans le produit cosmétique.

3. Produit cosmétique solide pour lèvres selon la revendication 2, dans lequel le composant (e) est presque dispersé dans le composant (c).

4. Produit cosmétique solide pour lèvres selon l'une quelconque des revendications 1 à 3, dans lequel le composant (b) comprend un ou plusieurs types de silicones de méthyle phényle choisi parmi le groupe consistant en le triméthyle pentaphényle trisiloxane, le diphényle diméthicone, le diphényl siloxyphényl triméthicone et le phényl triméthicone.

5. Produit cosmétique solide pour lèvres selon l'une quelconque des revendications 1 à 4, dans lequel le composant (c) comprend un ou plusieurs groups consistant en le dipentaérythrityle hexahydroxystéarate, l'huile de ricine, le glycéryle monoisostéarate, le polyglycéryle isostéarate, dans lequel le nombre molaire d'addition de glycérine est 4 à 10 et le nombre de résidus d'acide isostéariques est 1 à 4, PEG/PPG-36/41 dyméthyle éther, PEG/polybutylène glycole-52/32 diméthyle éther, pentaérythrityle tetra(éthylhexanoate/benzoate) et pentaérythrityle tetra(béhénate/benzoate/éthylhexanoate).

6. Produit cosmétique solide pour lèvres selon l'une quelconque des revendications 1 à 5, dans lequel le composant (a) en outre comprend un éster de dimère d'acide dilinoléique.

7. Produit cosmétique solide pour lèvres selon l'une quelconque des revendications 1 à 6, en outre comprenant un ou plusieurs types du composant (f) choisi parmi le groupe consistant en les oligomères d'oléfines, le néopentylglycol dicaprate, le tri(2-éthylhexanoate de glycéryle), le sorbitan sesquiisostéarate, le propylène glycol monostéarate, le cetyl PEG/PPG-10/1 diméthicone, le diglycéryl diisostéarate, le glycéril diisostéarate, le pentaérythrityle tetraéthylhexanoate, le squalane, la paraffine liquide, le triméthylolpropane triisostéarate, le triméthylolpropane triéthylhexanoate, le malate diisostéaryle, et le éthylhexanoate cétylique.

8. Produit cosmétique solide pour lèvres, comprenant (a) à (d) suivants:
(a) 5 à 30% en poids de polyisobutène hydrogéné,
(b) 30 à 70% en poids de triméthyle pentaphényle trisiloxane,
(c) 0,5 à 15% en poids d'un ou plusieurs types d'huiles choisies parmi le groupe consistant en le dipentaérythrityle hexahydroxystéarate, l'huile de ricine et le polyglycéryle isostéarate, dans lequel le nombre molaire d'addition de glycérine est 4 à 10 et le nombre de résidus d'acide isostéariques est 1 à 4, et
(d) 4 à 12% en poids de cire.

9. Produit cosmétique solide pour lèvres, comprenant (a) à (d) suivants:
(a) 5 à 30% en poids de polyisobutène hydrogéné,
(b) 30 à 70% en poids de diphényle diméthicone,
(c) 0,5 à 15% en poids d'un ou plusieurs types d'huiles choisies parmi le groupe consistant en le dipentaérythrityle hexahydroxystéarate, l'huile de ricine, le polyglycéryle isostéarate, dans lequel le nombre molaire d'addition de glycérine est 4 à 10 et le nombre de résidus d'acide isostéariques est 1 à 4, PEG/PPG-36/41 dyméthyle éther et le pentaérythrityle tetra(béhénate/benzoate/éthylhexanoate) et
(d) 4 à 12% en poids de cire.
